(19) Europäisches Patentamt
European Patent Office
Office européen des brevets

(11) EP 1 134 583 A1

(12) **EUROPEAN PATENT APPLICATION**

(43) Date of publication:
**19.09.2001 Bulletin 2001/38**

(51) Int Cl.[7]: **G01N 33/497**, A01C 1/02, C12M 1/34

(21) Application number: **00200990.0**

(22) Date of filing: **17.03.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Nederlandse Organisatie voor toegepast-natuurwetenschappelijk Onderzoek TNO**
**2628 VK Delft (NL)**

(72) Inventor: **The designation of the inventor has not yet been filed**

(74) Representative: **Ottevangers, Sietse Ulbe et al**
**Vereenigde,**
**Postbus 87930**
**2508 DH Den Haag (NL)**

(54) **Measuring metabolic rate changes**

(57) The invention relates to methods for measuring metabolic states or rates or changes therein, such as growth rates, dying rates, cell division, metabolite production, and other biological activities of organisms, in particular of small multi-cellular organisms, of seeds and seedlings and of micro-organisms, such as fungi, yeast, bacteria, plant or animal cells and cultures thereof. The invention provides a method for determining a change in metabolic rate of at least one organism comprising placing said organism or part thereof in a container and repeatedly or continually measuring the concentration of a metabolic gas in said container to determine changes in consumption or production of said gas by said organism wherein said gas concentration is determined without essentially affecting the concentration of said gas in said container.

EP 1 134 583 A1

## Description

The invention relates to methods for measuring metabolic states, metabolic rates and changes therein, such as growth rates, dying rates, cell division, metabolite production, and other biological activities of organisms, in particular of small multi-cellular organisms, of seeds and seedlings and of micro-organisms, such as fungi, yeast, bacteria, plant or animal cells and cultures thereof.

Biological activities of organisms are manifold and are often studied by the chemical, physical, physiological of morphological ways they manifest themselves. Culturing organisms, be it micro-organisms in cell culture, plants, or others, requires managing these biological activities, and managing these activities often requires measuring the underlying metabolic activities. As an example herein germination of seeds is discussed, however, the invention extends to culturing other organisms where similar approaches apply.

A new plant formed by sexual reproduction starts as an embryo within the developing seed, which arises from the ovule. When mature, the seed is the means by which the new individual is dispersed, though frequently the ovary wall or even extrafloral organs remain in close association to from a more complex dispersal unit as in grasses and cereals. The seed, therefore, occupies a critical position in the life history of the higher plant. The success with which the new individual is established -the time, the place, and the vigour of the young seedling- is largely determined by the physiological and biochemical features of the seed. Of key importance to this success are the seed's responses to the environment and the food reserves it contains, which are available to sustain the young plant in the early stages of growth before it becomes an independent, autotrophic organism, able to use light energy. People also depend on these activities for almost all of their utilisation of plants.

Cultivation of most crop species depends on seed germination, though, of course, there are exceptions when propagation is carried out vegetatively. Moreover, seed such as those of cereals and legumes are themselves major food sources whose importance lies in the storage reserves of protein, starch, and oil laid down during development and maturation.

In the scientific literature the term germination is often used loosely and sometimes incorrectly and so it is important to clarify its meaning. Germination begins with water uptake by the seed (imbibition) and ends with the start of elongation by the embryonic axis, usually the radicle. It includes numerous events, e.g., protein hydration, subcellular structural changes, respiration, macromolecular syntheses, and cell elongation, none of which is itself unique to germination. But their combined effect is to transform an organism having a dehydrated, resting metabolism into an organism having an active metabolism, culminating in growth.

Germination sensu stricto therefore does not include seedling growth, which commences when germination finishes. Hence, it is incorrect, for example, to equate germination with seedling emergence from soil since germination will have ended sometime before the seedling is visible. Seed testers often refer to germination in this sense because their interests lie in monitoring the establishment of a vigorous plant of agronomic value. However, physiologists do not encourage such a definition of the term germination but in general acknowledge its widespread use by seed technologists. It would however be preferable to find a more defined definition. Processes occurring in the nascent seedling, such as mobilisation of the major storage reserves, are also not part of germination: they are postgermination events.

A seed in which none of the germination processes is taking place is said to be quiescent. Quiescent seeds are resting organs, generally having a low moisture content (5-15%) with metabolic activity almost at a standstill. A remarkable property of seeds is that they are able to survive in this state, often for many years, and subsequently resume a normal, high level of metabolism. For germination to occur quiescent seeds generally need only to be hydrated under conditions that encourage metabolism, e.g., a suitable temperature and presence of oxygen.

Components of the germination process, however, may occur in a seed that does not achieve radicle emergence. Even when conditions are apparently favourable for germination so that imbibition, respiration, synthesis of nucleic acids and proteins, and a host of other metabolic events all proceed, culmination in cell elongation does not occur, for reasons that are still poorly understood; such a seed expresses dormancy. Seeds that are dispersed from the parent plant already containing a block to the completion of germination show primary dormancy. Sometimes, a block(s) to germination develops in hydrated, mature seeds when they experience certain environmental conditions, and such seeds show induced or secondary dormancy. Dormant seeds are converted into germinable seeds (i.e., dormancy is broken) by certain "priming" treatments such as a light stimulus or a period at low or alternating temperature which nullify the block to germination but which themselves are not needed for the duration of germination process.

The extent to which germination has progressed can be determined roughly, say by measuring water uptake or respiration, but these measurements give us only a very broad indication of what stage of the germination process has been reached. No universally useful biochemical marker of the progress of germination has been found. The only stage of germination that we can time fairly precisely is its termination. Emergence of the axis (usually the radicle) from the seed normally enables us to recognise when germination has gone to completion, though in those cases where the axis may grow before it penetrates through the surrounding tissues, the completion of germination can be determined as the time when a sustained rise in fresh weight begins.

We are generally interested in following the germination behaviour of large numbers of seeds, e.g., all the seeds produced by one plant or inflorescence, or all those collected in a soil sample, or all those subjected to certain experimental treatment. The degree to which germination has been completed in a population is usually expressed as a percentage, normally determined at time intervals over the course of the germination period which can be expressed in so-called germination curves, about which some general points should be made. Germination curves are usually sigmoidal, a minority of the seeds in the population germinates early, then the germination percentage increases more or less rapidly, and finally few late germinatores emerge. The curves are often positively skewed because a greater percentage germinates in the first half of the germination period than in the second. But although the curves have the same general shape, important differences in behaviour between populations are evident. For example, curves often flatten off when only a low percentage of the seeds has germinated, showing that this population has germination capacity, i.e., the proportion of seeds capable of completing germination is low. Assuming that these seeds are viable, the behaviour of the population could be related to dormancy or to environmental conditions, such as temperature or light, which do not favour germination of most of the seeds.

The shape of the curves also depends on the uniformity of the population, i.e., the degree of simultaneity or synchrony of germination. When a limited percentage of seeds succeeds in germinating fairly early, but the remainder begin to do so only after a delay the population seems to consists of two discrete groups: the quick and the slow germinators. This example also illustrates the point that populations with the same germination capacity can differ in other respects. Three respiratory pathways are assumed to be active in the imbibed seed: glycolysis, the pentose phosphate pathway, and the citric acid (Krebs or tricarboyxlic acid) cycle. Glycolysis, catalysed by cytoplasmic enzymes, operates under aerobic and anaerobic condition to produce pyruvate, but in the absence of $O_2$ this is reduced further to ethanol, plus $CO_2$, or to lactic acid if no decarboxylation occurs. Anaerobic respiration, also called fermentation, produces only two ATP molecules per molecule of glucose respired, in contrast to six ATPs produced during pyruvate formation under aerobic conditions. In the presence of $O_2$, further utilisation of pyruvate occurs within the mitochondrion: oxidative decarboxylation of pyruvate produces acetyl-CoA, which is completely oxidised to $CO_2$ and water via the citric acid cycle to yield up to a further 30 ATP molecules per glucose molecule respired. The generation of ATP molecules occurs during oxidative phosphorylation when electrons are transferred to molecular $O_2$ along an electron transport (redox) chain via a series of electron carriers (cytochromes) located on the inner membrane of the mitochondrion. An alternative pathway for electron transport, which does not involve cytochromes, may also operate in mitochondria.

The pentose phosphate pathway is an important source of NADPH, which serves as a hydrogen and electron donor in reductive biosynthesis, especially of fatty acid. Intermediates in this pathway are starting compounds for various biosynthetic processes, e.g., synthesis of various aromatics and perhaps nucleotides and nucleic acid. Moreover, complete oxidation of hexose via the pentose phosphate pathway and the citric acid cycle can yield up to 29 ATPs.

Respiration by mature "dry" seeds (usual moisture content: 10-15%) of course is extremely low when compared with developing or germinating seeds, and often measurements are confounded by the presence of a contaminating microflora. When dry seeds are introduced to water ,these is an immediate release of gas. This so-called "wetting burst" which may last for several minutes, is not related to respiration, but is the gas that is released from colloidal adsorption as water is imbibed. This gas is released also when dead seeds or their contents, e.g., starch, are imbibed.

Keto acids (e.g.; $\alpha$-ketoglutarate, pyruvate), which are important intermediates in respiratory pathways, are chemically unstable and may be absent from the dry seed. A very early metabolic events during imbibition, occurring within the first few minutes after water enters the cells, is their reformation from amino acids by deamination and transamination reactions (e.g., of glutamic acid and alanine).

The consumption of $O_2$ by many seeds follows a basic pattern although the pattern of consumption by the embryo differs ultimately from that by storage tissues. Respiration is considered to involve three or four phases:

Phase 1. Initially there is a sharp increase in $O_2$ consumption, which can be attributed in part to the activation and hydration of mitochondrial enzymes involved in the citric acid cycle and electron transport chain. Respiration during this phase increases linearly with the extent of hydration of the tissue.

Phase 2. This is characterised by a lag in respiration as $O_2$ uptake is stabilised or increases only slowly. Hydration of the seed parts is now completed and all pre-existing enzymes are activated. Presumably there is little further increase in respiratory enzymes or in the number of mitochondria during this phase. The lag phase in some seeds may occur in part because the coats or other surrounding structures limit $O_2$ uptake to the imbibed embryo or storage tissues, leading temporarily to partially anaerobic conditions. Removal of the testa from imbibed pea seeds, for example, diminishes the lag phase appreciably. Another possible reason for this lag is that the activation of the glycolytic pathway during germination is more rapid than the development of mitochondria. This could lead to an accumulation of pyruvate because of deficiencies in the citric acid cycle or oxidative phosphorylation (electron transport chain); hence, some pyruvate would be diverted temporarily to the fermentation pathway, which is not $O_2$ requiring.

Between phase 2 and 3 in the embryo the radicle penetrates the surrounding structures: germination is completed.

Phase 3. There is now a second respiratory burst. In the embryo, this can be attributed to an increase in activity of newly synthesised mitochondria and respiratory enzymes in the proliferating cells of the growing axis. The number of mitochondria in storage tissues also increases, often in association with the mobilisation of reserves. Another contributory factor of the rise in respiration in both seeds parts could be an increased $O_2$ supply through the now punctured testa (or other surrounding structures).

Phase 4. This occurs only in storage tissues and coincides with their senescence following depletion of the stored reserves.

The lengths of phases 1-4 vary from species to species owing to such factors as differences in rates of imbibition, seed-coat permeability to oxygen, and metabolic rates. Moreover, the lengths of the phases will vary considerably with the ambient conditions, especially the temperature. In a few seeds, e.g., Avena fatua, there is no obvious lag phase in oxygen uptake. The reasons for its absence are not known, but it could be because efficient respiratory systems become established early following imbibition, including the development of newly active mitochondria, thus ensuring a continued increase in $O_2$ utilisation. Also, coat impermeability might not restrict $O_2$ uptake prior to the completion of germination.

During germination a readily available supply of substrate for respiration must be present. This may be provided to a limited extent by hydrolysis of the major reserves, e.g., triacylgylcerols, which are present in almost all parts of the embryo, including the radicle and hypocotyl, although their greatest concentration is in storage tissues. It is important to note, however, that extensive mobilisation of reserves is a postgerminative event. Most dry seeds contain sucrose, and many contain one or more of the raffinose-series oligosaccharides: raffinose (galactosyl sucrose), stachyose (digalactosyl sucrose), and verbascose (trigalactosyl sucrose), although the latter is usually present only as a minor component. The distribution and amounts of these sugars within seeds are very variable, even between different varieties of the same species.

During germination, sucrose and the raffinose-series oligosaccharides are hydrolysed, and in several species the activity of $\alpha$-galactosiidase, which cleaves the galactose units from the sucrose, increases as raffinose and stachyose decline. Although there is little direct evidence that the released monosaccharides are utilised as respiratory substrates, there is strong circumstantial evidence. Free fructose and glucose may accumulate in seeds during the hydrolysis of sucrose and the oliosaccharides, but there is no build-up of galactose (e.g., in mustard, Sinapis alba). Hence, it is probably rapidly utilised, perhaps through incorporation into cell walls or into galactolipids of the newly forming membranes in the cells of developing seedling.

Virtually all metabolic pathways in living organisms, and not only those related to germination, relate to the uptake or release of metabolic gasses, of which the two most important are oxygen and carbon dioxide; examples of others are carbon mono-oxide, nitric oxide, nitric dioxide, dinitric oxide, ethylene and ethanol. Classical is the way it could be demonstrated that oxygen is central to life. A mouse, placed under a glass bulb together with a burning candle, died when the flame dwindled and died, showing that also the mouse could not do without the oxygen. Undoubtedly, the level of carbon dioxide in the glass bulb was, as a consequence, high.

The above example illustrates an archaic way of measuring the underlying metabolic activity of an organism. More modern methods have been developed which comprise measuring oxygen or other metabolic gasses in gas or liquid media. Oxygen, or other gasses, in gas are often measured by analysis with gas-chromatography. In liquid gas contents are often measured by flushing some liquid through an electrochemical measurement device.

For both types of measurements the sample is in general spilled and cannot be reused for other measurements. This has a number of further disadvantages: A container with the organism under study has to be opened for a gas determination, which may disrupt the activities to be measured or otherwise hinder accurate determination. Also, for each point in a time series different samples are necessary for which the container has to be opened again. Normally this makes the number of samples very large and does not allow for using small containers to begin with. Furthermore, sample to sample variability makes it very difficult to get reliable figures and the costs for handling and measuring a sample are in general very high.

The invention provides a method for determining the metabolic state of at least one organism or part thereof comprising placing said organism or part thereof in a container and measuring the concentration of a metabolic gas in said container to determine consumption or production of said gas by said organism or part thereof wherein said gas concentration is determined without essentially affecting the concentration of said gas in said container. If no or no change in metabolic gasses are detected (in practice for a sufficiently long period), it may for example be assumed that the organism is dead or in a hibernating state, in particular now where the invention provides that no gas is consumed by measuring, all changes in gas concentration must thus be attributed to the production and/or consumption of a metabolic gas, thus of life, or at least in a state of lifelike activity.

In a preferred embodiment, the invention provides a method for determining a change in metabolic state or rate of at least one organism or part thereof comprising placing said organism or part thereof in a container and repeatedly or continually measuring the concentration of a metabolic gas in said container to determine changes in consumption or production of said gas by said organism or part thereof wherein said gas concentration is determined without essentially affecting the concentration of said gas in said container. In one example of the invention one or more seeds are brought in a small container, along with some water to induce the germination process. Seeds can of course be totally immersed in water, which typically allows for measurements to be made in the liquid but usually measurement of the air or gas above the seeds will be sufficient. When desired the container is a closed or confined space that is essentially not opened during measurements but to which additional substances (oxygen, nutrients, growth hormones, etc.) can be added with for example a valve or injection system. Due to the germination at a certain point in time the seed(s) will start to consume oxygen and produce carbon dioxide. The oxygen concentration will drop from the moment the germination starts and the carbon dioxide concentration will rise. The gas concentration is preferably measured optically. This can for example be achieved by a measuring device which is at least partly set up within the container, but measurements can also be made through a clear portion of the wall of the container, which for example could be made of glass.

In one embodiment, the invention provides a optical method based on fluorescence quenching of fluorescent compounds by oxygen (1,2,3,4), to determine the oxygen levels inside a container, preferably without opening it. A sample can be measured over and over again in the time, and is not destroyed. Moreover, because the sample is not destroyed the number of samples necessary to do a time study is considerably lower compared to conventional methods. In a preferred embodiment, the invention provides a method wherein said gas concentration is determined by determining the fluorescence quenching of a fluorescent dye, preferably a suitable organo-metal, present in said container. For measuring oxygen, an oxygen sensitive dye such as a ruthenium bipyridyl complex, or Tris-$Ru^{2+}$ 4,7 biphenyl 1,10 phenantrolin; or another Ru(ruthenium)-complex, or another organo-metal complex, such as an Os-complex or a Pt-complex, is suitable, for measuring carbon dioxide, or other gasses such as CO, NO, NO2, N2O, ethylene or ethanol, suitable sensitive organo-metal dyes, such as tris[2-(2-pyrazinyl)thiazole] ruthenium II (5) are used

For example, the optical oxygen sensing measurement technique used herein is based on the fluorescence quenching of a metal organic fluorescent dye. The dye which is very sensitive to oxygen, is for example excited by a short laser light-pulse of for example 1 microsecond. After the excitation has stopped the oxygen sensitive dye emits fluorescent light with a decay curve which depends on the oxygen concentration. The process behind this phenomenon is called dynamic quenching.

Preferably said dye is present in a gas permeable compound such as silica or a hydrophobic polymer such as a (optionally fluoridated) silicone polymer, in PDMS (polydimethylsiloxane), in PTMSP (polytrimethylsilylpropyl), or in a mixture thereof but of course it can be contained in other suitable compounds as well. In a preferred embodiment the invention provides a method wherein said dye is present in at least a part of an inner coating of said container, for example situated on the inside of an optically transparent part of the container when measurement is from the outside.

Measuring can for example be achieved by measuring the fluorescence lifetime. The excited molecules are deactivated by oxygen in a collision process. The quenching process does not consume the gas (here the oxygen) so liquid medium does not necessarily have to be stirred to obtain the measurements. The fluorescence lifetime gets shorter because the probability of the molecules to be deactivated gets higher for molecules which stay longer in the excited state. The effect is proportional with the quencher concentration. The relation between fluorescence lifetime and gas (here oxygen) concentration is given by the Stern Volmer equation (1)

$$\frac{\tau_0}{\tau} = 1 + C_{SV} * [O_2]$$

where $\tau_0$ is the fluorescence lifetime at quencher ($O_2$) concentration zero, $\tau$ is the fluorescence lifetime at a specific quencher ($O_2$) concentration. $C_{SV}$ is the Stern-Volmer constant and $[O_2]$ is the gas concentration.

Measuring can also be achieved by measuring the fluorescence intensity. The fluorescent compound is excited by a continuously radiating light source such as a LED and the fluorescence intensity is measured. More gas (here oxygen) caused less fluorescence. The relation between the oxygen concentration and the intensity is given by the Stern Volmer equation (2)

$$\frac{I_0}{I} = 1 + C_{SV} * [O_2]$$

where $I_0$ is the fluorescence intensity at quencher ($O_2$) concentration zero, I is the fluorescence intensity at a specific quencher ($O_2$) concentration. $C_{SV}$ is the Stern-Volmer constant and $[O_2]$ is the gas concentration.

Using the fluorescence lifetime method has the advantage that the measurement is independent of the source intensity, detector efficiency, fluorescent probe concentration etc. A method based on this principle is robust and less prone to drift. Moreover, because the quenching process does not consume oxygen or other metabolic gasses, the method as provided by the invention is very useful to measure metabolic rate changes of organisms by measuring an increase or decrease in metabolic gas production or consumption by said organism or organisms.

A method as provided by the invention is based on a time gated measurement (Fig.1). In this measurement method the fluorescence is determined in two time windows (A and B) after a light pulse. Fluorescence lifetime is a function of the ratio between A and B and is proportional to the oxygen concentration. Figure 2 shows an instrumental set up. A light source (e.g. LED or laser) is pulsed, the light pulses are filtered and excite the fluorescent dye located in the environment where the metabolic gas has to be determined. The resulting fluorescence response is detected in a detector, the information is digitised, if needed the measurement is corrected (for temperature for example) and the gas concentration is calculated and displayed.

In the detailed description an example of a method according to the invention is shown wherein said organism comprises a seed, and wherein said change in metabolic rate denotes germination. However, a method according to the invention is as well applicable to register a second respiratory burst as is often identified in phase 3 of germination. Of course, the invention provides as well a method wherein said organism or part thereof comprises one or more micro-organisms or part thereof such as a protoplast, plastid (e.g. chloroplast) or mitochondrium, comprises plant cell cultures, comprises plant tissue explants, whole plants or seedlings, parts or organs of plants such as flowers, leaves, stems, roots, sexual organs, tubers, bulbs, fruits, or comprises animal cell cultures, animal tissue explants, parts or organs of animals, blood, comprises a bacterium or bacterial cultures, a yeast cell or yeast cultures, a fungus or fungal cultures, and so on.

The invention provides a method wherein said change in metabolic rate denotes cell activity of said organism or part thereof or micro-organism or cultures thereof, or, alternatively, wherein said change in metabolic rate denotes cell death, and to detect circumstances wherein such cell-activities thrive, or not. A method according to the invention is for example useful to detect ( the onset of) sporulation of bacterial cultures, or microbial fermentation.

Within the field of seed technology, the invention provides a method to determine or monitor a rate of seed germination or development, to for example determine proper priming of seed batches. Therewith, the invention also provides a seed batch monitored with a method according to the invention. Said seed batches have accurately been primed. Similarly, the invention provides a method to determine or monitor a rate of culture development of a cell- or tissue-culture comprising use of a method according to the invention and a cell- or tissue culture monitored with a method according to the invention. Other methods provides for example entail a method to determine or monitor processing of waste water comprising use of a method according to the invention, or other processes where micro-biological fermentation plays a role. The invention is further explained in the detailed description without limiting the invention thereto.

Detailed description.

Determination of the start of seed germination by oxygen consumption

Most of conventional agriculture is engaged in growing plants from seeds. Plant breeding programs are dependent on the germination of the seeds obtained. Therefore the slow or no germination of seeds has a major impact on food production and research. The research on dormancy or environmental factors influencing the germination requires a simple method to monitor the germination process.

Methods.

One or more seeds are brought in a container, along with some water to induce the germination process. The container is closed. Due to the germination at a certain point in time the seed(s) will start to consume oxygen. Because the container is closed, the oxygen concentration will drop from the moment the germination starts. This can be monitored with a special oxygen sensitive coating on the inside of an optically transparent part of the container. An advantage of optical oxygen determination is the fact that the coating itself does not consume any oxygen. In this way the start of the germination can be monitored accurately. Up to now only the appearance of a root was an indication of the germination. In the experiment the first root showed after 10 to 14 hours. From the oxygen measurements we see that the germination activity showed after 3.5 hours. The oxygen consumption is an early indicator of seed germination.

The point where the germination starts can be calculated from the measured oxygen levels, as for example given in table 1. The linear extrapolation of the oxygen levels measured after 4 hours in the containers with 1, 2 and 3 seeds show an intersection with the oxygen level of an empty container at 3.5 hours after the addition of the water. This is the point in time where the metabolism of the germination starts. This is shown in figure 3. Figure 4 shows a general course of oxygen levels with seeds in a container and figure 5 an example of a calculation. With this method it is possible to examine the effect of all kind of environmental influences on the germination process. It also gives an opportunity to influence the germination process in an early stage. This method is a simple and powerful tool in germination research and in the priming of seeds.

Table 1

| Oxygen content in µg of a sample container (approx. 1 ml) with a different number of seeds. | | | |
|---|---|---|---|
| Hours | Container with 1 seed | Container with 2 seeds | Container with 3 seeds |
| 0.0 | 284 | 284 | 284 |
| 0.5 | 263 | 271 | 261 |
| 1.5 | 274 | 253 | 261 |
| 2.5 | 284 | 279 | 267 |
| 3.5 | 274 | 261 | 270 |
| 4.5 | 270 | 259 | 266 |
| 5.5 | 279 | 256 | 223 |
| 6.5 | 277 | 253 | 227 |
| 7.5 | 271 | 231 | 204 |
| 8.5 | 282 | 257 | 198 |
| 9.5 | 269 | 227 | 160 |
| 11.5 | 275 | 202 | 125 |
| 14.0 | 262 | 172 | 51 |
| 24.0 | 220 | 41 | 1 |
| 29.0 | 250 | 14 | 7 |
| 32.0 | 238 | 9 | 4 |
| 35.5 | 226 | 8 | 4 |
| 38.0 | 201 | 8 | 4 |
| 50.0 | 171 | 6 | 5 |
| 53.0 | 191 | 8 | 5 |
| 61.0 | 159 | 9 | 4 |
| 72.0 | 135 | 7 | 3 |

Figure legends

Figure 1 Measurement principle of optical oxygen sensor.
Figure 2 Schematic representation of oxygen sensor.
Figure 3 Oxygen consumption during the germination of Triumph 1989 seeds at 25 °C.
Figure 4 Oxygen consumption during the germination of seeds.
Figure 5 Regression calculation.

References

1. Bambot S.B. et al., Phase Fluorimetric Sterilizable Optical Oxygen Sensor, Biotechnology and Bioengineering, 43:1139-1145, 1994.
2. Cox, M.E., Bunn, B., Detection of Oxygen by fluorescence quenching, Applied Optics, 24, 2114--2120, 1985
3. Holst, G.A., Flox an oxygen-flux-measuring system using a phase modulation method to evaluate the oxygen dependent fluorescent lifetime, Sensor and Actuators B 29 (1995) 231-239
4. Meier, B et al., Novel oxygen sensor material based on a ruthenium bipyridyl complex encapsulated in zeolyte Y: dramatic diffeneces in the efficience of luminescence quenching by oxygen on going from surface-adsorbed to zeolite-encapsulated fluorophores, Sensor and Actuators B 29 (1995) 240-245.
5. Marazuele, M.D. et al., Luminescence lifetime Quenching of a Ruthenimum (II) Polypyridyl Dye for Optical Sensing of Carbon Dioxide. Appl. Spectrocospy (1998), 52:1314-1320.

## Claims

1. A method for determining metabolic state or rate or a change therein of at least one organism or part thereof comprising placing said organism or part thereof in a container and measuring the concentration of a metabolic gas in said container to determine consumption or production of said gas by said organism or part thereof wherein said gas concentration is determined without essentially affecting the concentration of said gas in said container.

2. A method according to claim 1 wherein said container is essentially not opened during measurements.

3. A method according to claim 1 or 2 wherein said gas comprises oxygen.

4. A method according to anyone of claims 1 to 3 wherein said gas concentration is determined optically.

5. A method according to claim 4 wherein said gas concentration is determined by determining the fluorescence quenching of a fluorescent dye present in said container.

6. A method according to claim 5 wherein said dye is present in a gas permeable compound.

7. A method according to claim 6 wherein said compound comprises a hydrophobic polymer.

8. A method according to anyone of claims 5 to 7 wherein said dye is present in at least a part of an inner coating of said container.

9. A method according to anyone of claims 1 to 8 wherein said organism comprises a seed.

10. A method according to claim 9 wherein said change in metabolic rate denotes germination.

11. A method according to anyone of claims 1 to 8 wherein said organism comprises a micro-organism.

12. A method according to claim 11 wherein said change in metabolic rate denotes cell activity.

13. A method according to claim 11 wherein said change in metabolic rate denotes cell death.

14. A method according to claim 11 wherein said change denotes sporulation.

15. A method according to claim 11 wherein said change denotes microbial fermentation.

16. A method to determine or monitor a rate of seed development comprising use of a method according to claim 9 or 10.

17. A seed batch monitored with a method according to claim 16.

18. A method to determine or monitor a rate of culture development of a cell- or tissue-culture comprising use of a method according to anyone of claims 11 to 15.

19. A cell- or tissue culture monitored with a method according to claim 18.

20. A method to determine or monitor processing of waste water comprising use of a method according to anyone of claims 1 to 8.

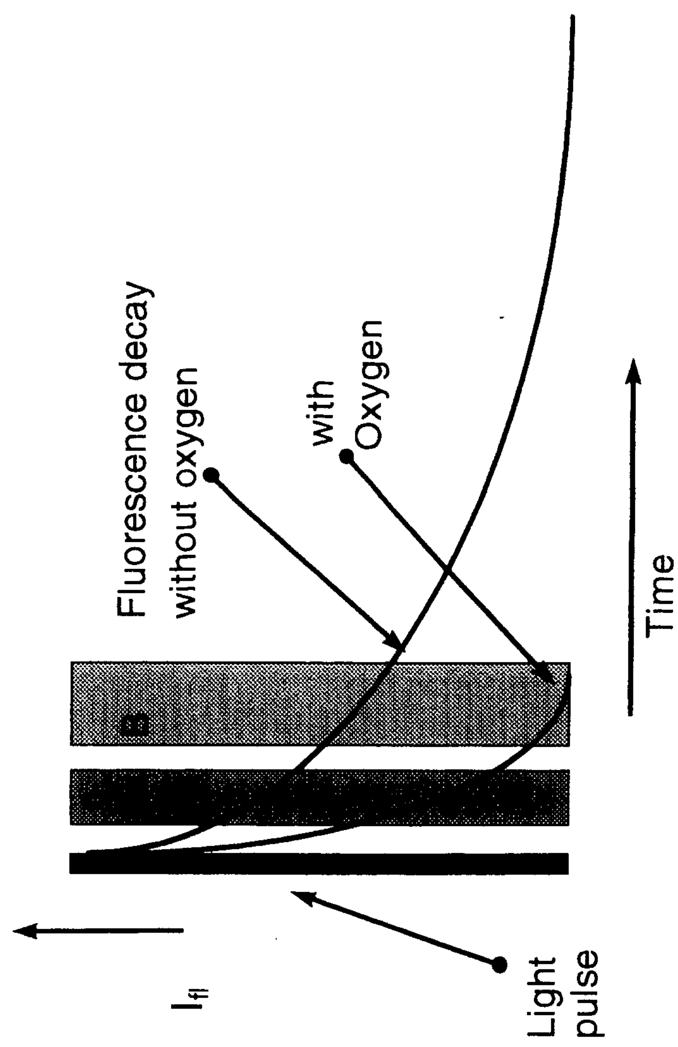
Fluorescence decay
without oxygen
with
Oxygen
Time
$I_{fl}$
Light
pulse

**Figure 1**

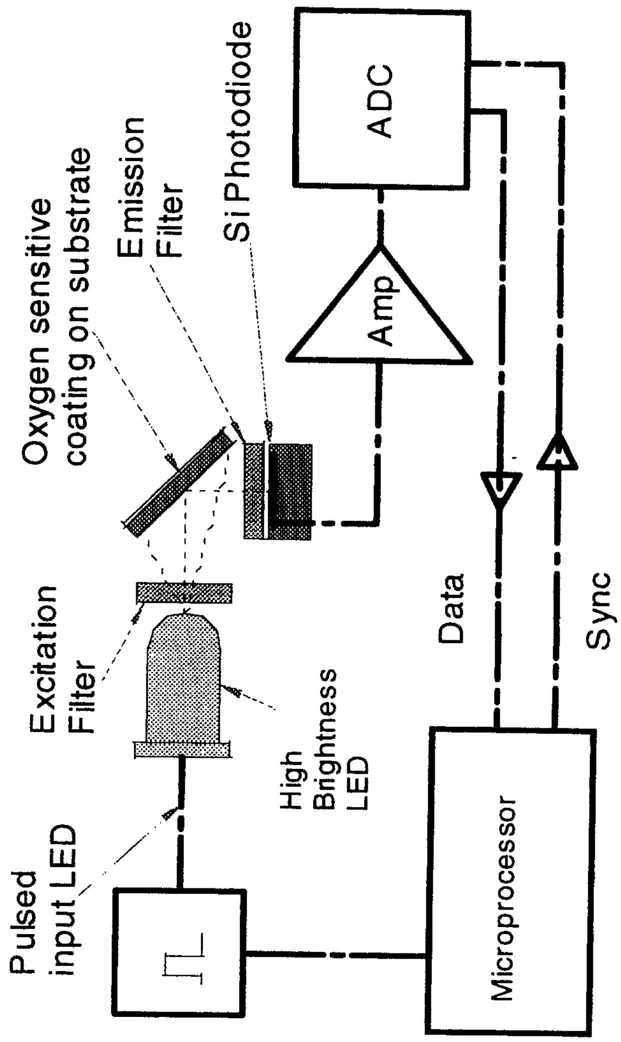
Oxygen sensitive
coating on substrate
Emission
Filter
Si Photodiode
ADC
Amp
Excitation
Filter
High
Brightness
LED
Pulsed
input LED
Data
Sync
Microprocessor

Figure 2

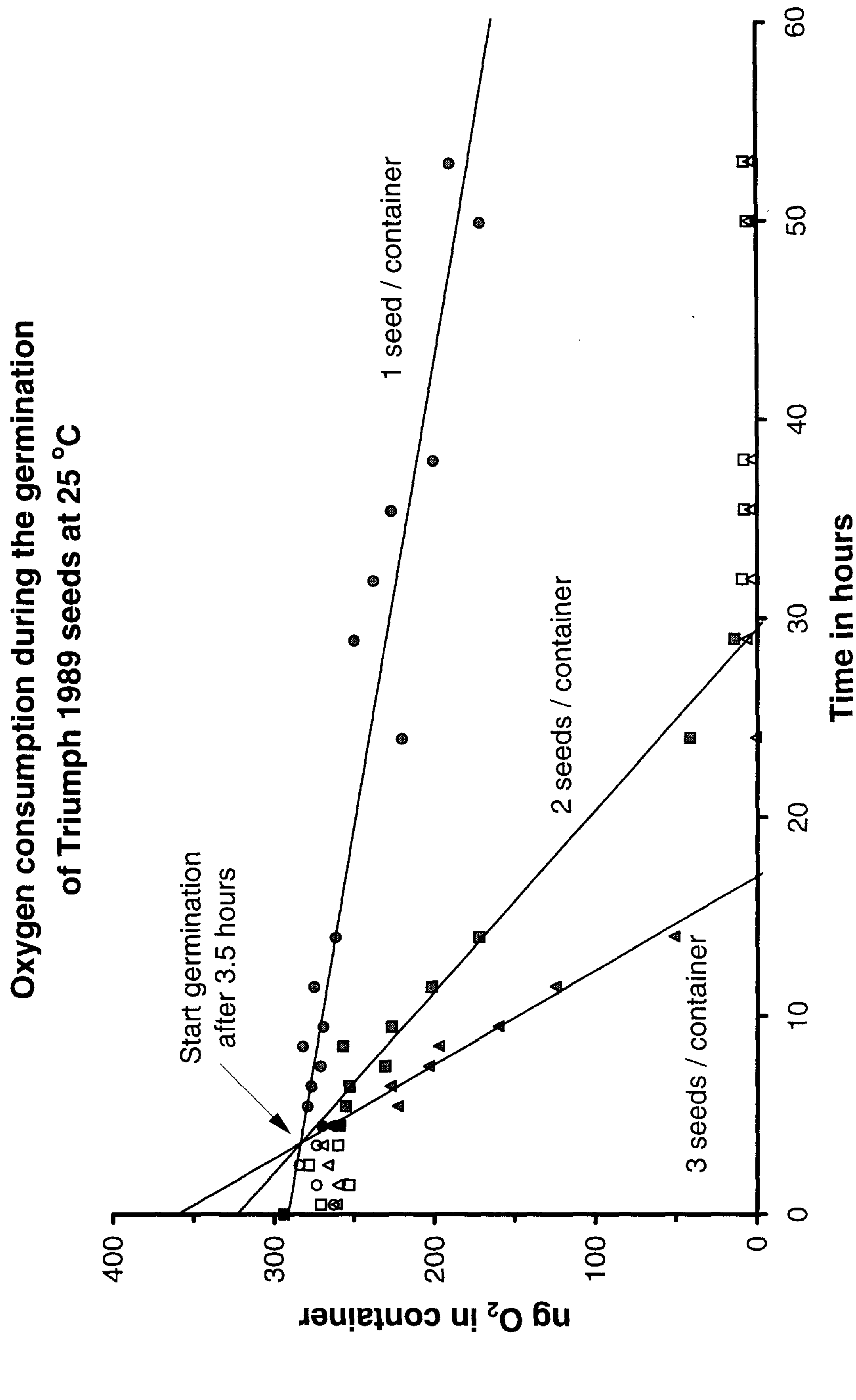
Oxygen consumption during the germination
of Triumph 1989 seeds at 25 °C
Start germination
after 3.5 hours
1 seed / container
2 seeds / container
3 seeds / container
ng $O_2$ in container
400
300
200
100
0
Time in hours
0
10
20
30
40
50
60

FIGURE 3

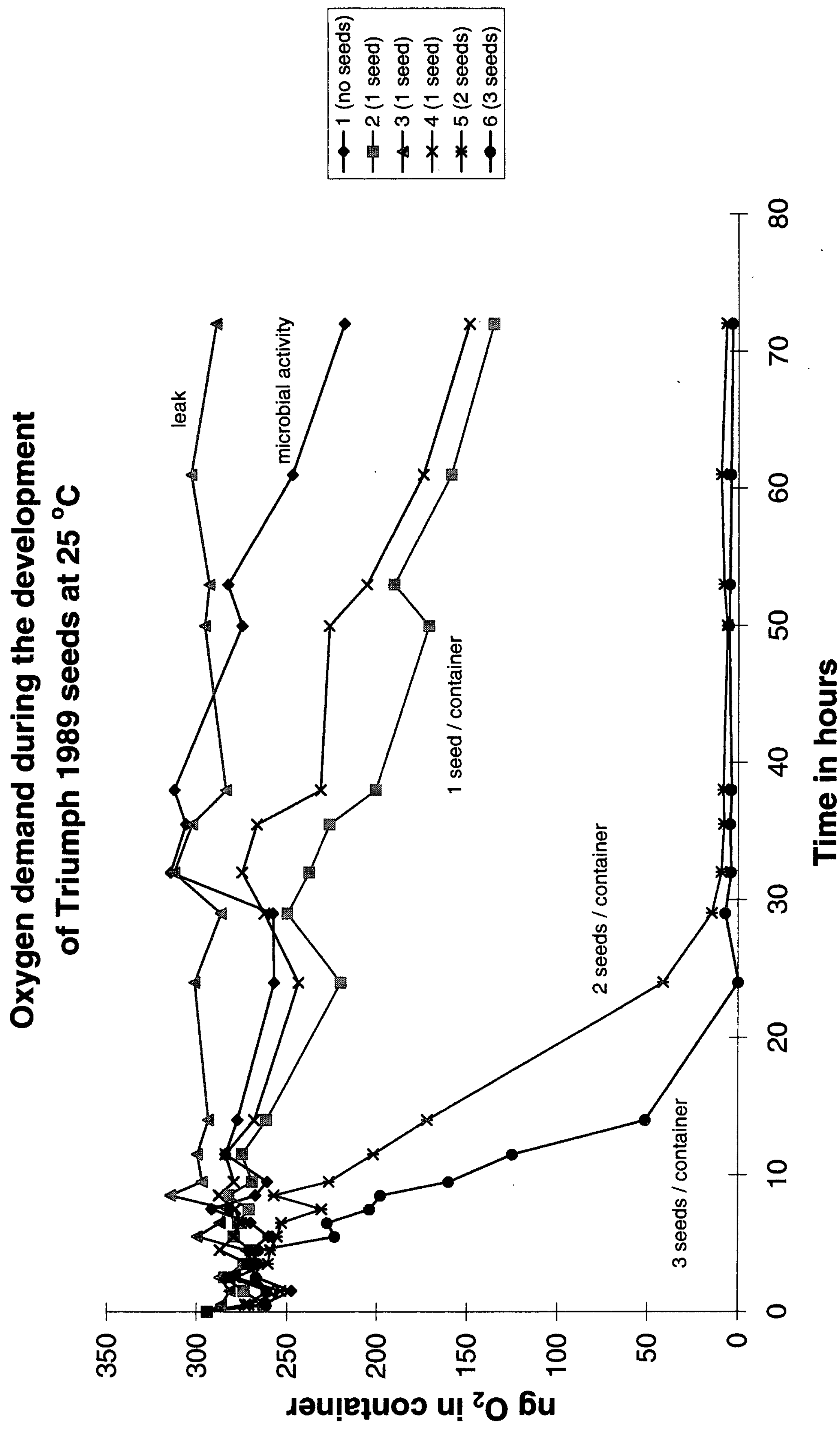

Oxygen demand during the development of Triumph 1989 seeds at 25 °C
1 (no seeds)
2 (1 seed)
3 (1 seed)
4 (1 seed)
5 (2 seeds)
6 (3 seeds)
leak
microbial activity
1 seed / container
2 seeds / container
3 seeds / container
ng $O_2$ in container
350
300
250
200
150
100
50
0
0
10
20
30
40
50
60
70
80
Time in hours


FIGURE 4

Calculation start

| Hours | 2 (1 seed) | 4 (1 seed) | 5 (2 seeds) | 6 (3 seeds) |
|---|---|---|---|---|
| 5.5 | 279 | 279 | 256 | 223 |
| 6.5 | 277 | 275 | 253 | 227 |
| 7.5 | 271 | 279 | 231 | 204 |
| 8.5 | 282 | 287 | 257 | 198 |
| 9.5 | 269 | 279 | 227 | 160 |
| 11.5 | 275 | 284 | 202 | 125 |
| 14.0 | 262 | 268 | 172 | |
| 24.0 | 220 | 244 | | |
| 29.0 | 250 | 263 | | |
| 32.0 | 238 | 275 | | |
| 35.5 | 226 | 267 | | |
| 38.0 | 201 | 231 | | |

| Hours | 2 (1 seed) | 4 (1 seed) | 5 (2 seeds) | 6 (3 seeds) |
|---|---|---|---|---|
| 5.5 | 0 | 0 | 23 | 56 |
| 6.5 | 2 | 4 | 26 | 52 |
| 7.5 | 8 | 0 | 48 | 75 |
| 8.5 | -3 | -8 | 22 | 81 |
| 9.5 | 10 | 0 | 52 | 119 |
| 11.5 | 4 | -5 | 77 | 154 |
| 14.0 | 17 | 11 | 107 | |
| 24.0 | 59 | 35 | | |
| 29.0 | 29 | 16 | | |
| 32.0 | 41 | 4 | | |
| 35.5 | 53 | 12 | | |
| 38.0 | 78 | 48 | | |

| | | | | |
|---|---|---|---|---|
| r.c. | 2.001 | 0.950 | 9.948 | 17.747 |
| intercept | -12.083 | -7.808 | -38.721 | -55.444 |
| start hour | 6.0 | 8.2 | 3.9 | 3.1 |

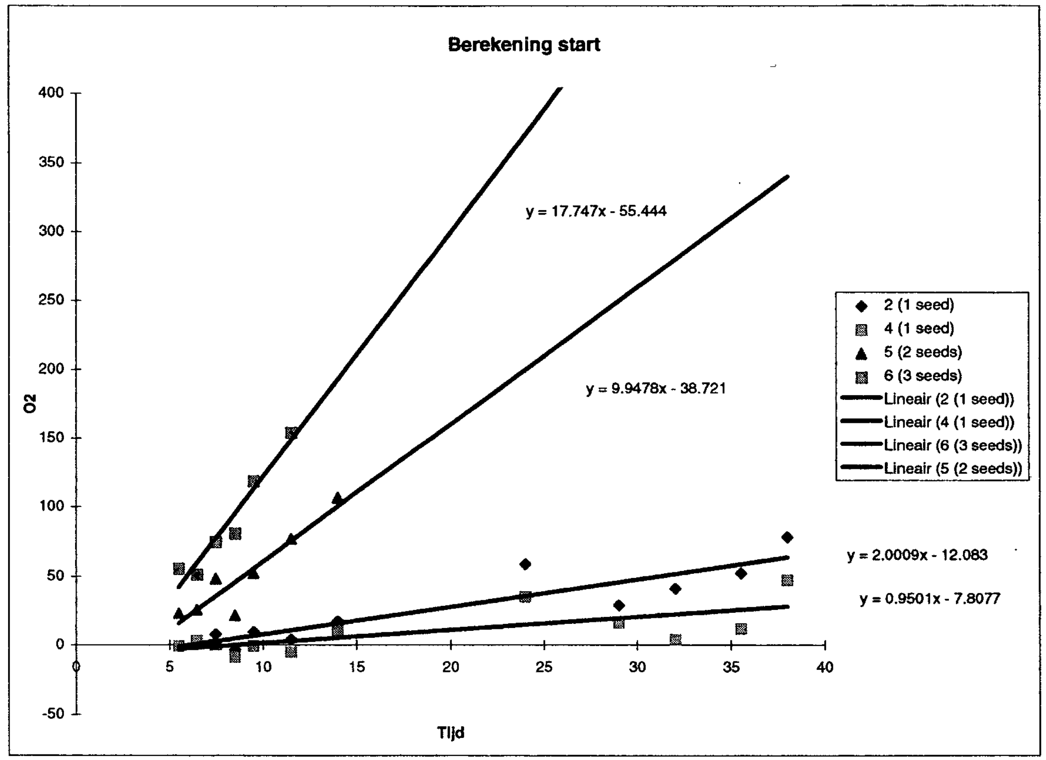


FIGURE 5

European Patent Office

# EUROPEAN SEARCH REPORT

Application Number
EP 00 20 0990

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br><br>Y | WO 98 54354 A (UNIV PENNSYLVANIA)<br>3 December 1998 (1998-12-03)<br><br>* the whole document * | 1-5,11,<br>12,15,<br>18-20<br>6,7,9,<br>10,16,<br>17,20 | G01N33/497<br>A01C1/02<br>C12M1/34 |
| X | WO 98 12348 A (BECTON DICKINSON CO ;BURRELL GREGORY J (US); HU JOANNA KWOK YU (US) 26 March 1998 (1998-03-26)<br>* the whole document * | 1-5,11,<br>12,18,19 | |
| Y | US 5 863 460 A (SLOVACEK RUDOLF E ET AL)<br>26 January 1999 (1999-01-26)<br>* abstract; figures * | 6,7 | |
| Y | WO 96 36875 A (TEMPLETON COLIN W G ;COLOMBO STEPHEN J (CA))<br>21 November 1996 (1996-11-21)<br>* page 22, line 6 - page 26, line 4 * | 9,10,16,<br>17 | |
| | | | **TECHNICAL FIELDS SEARCHED (Int.Cl.7)** |
| Y | AN L ET AL: "A NEW BIOSENSOR FOR RAPID OXYGEN DEMAND MEASUREMENT"<br>WATER ENVIRONMENT RESEARCH,US,WATER ENVIRONMENT FEDERATION, ALEXANDRIA,<br>vol. 70, no. 5, 1 July 1998 (1998-07-01), pages 1070-1074, XP000793304<br>ISSN: 1061-4303<br>* page 1073, paragraph 3 * | 20 | G01N<br>A01C<br>C12M |
| D,A | COX M E ET AL: "DETECTION OF OXYGEN BY FLUORESCENCE QUENCHING"<br>APPLIED OPTICS,US,OPTICAL SOCIETY OF AMERICA,WASHINGTON,<br>vol. 24, no. 14, 1985, pages 2114-2120, XP000885259<br>ISSN: 0003-6935<br>* the whole document *<br><br>-/-- | 1-20 | |

The present search report has been drawn up for all claims

| Place of search | Date of completion of the search | Examiner |
|---|---|---|
| THE HAGUE | 20 September 2000 | Bosma, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 20 0990

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | GIDROL X ET AL: "ACCUMULATION OF REACTIVE OXYGEN SPECIES AND OXIDATION OF CYTOKININ IN GERMINATING SOYBEAN SEEDS" EUROPEAN JOURNAL OF BIOCHEMISTRY,DE,BERLIN, vol. 224, no. 1, 15 August 1994 (1994-08-15), pages 21-28, XP000610708 ISSN: 0014-2956 * abstract * --- | 9,10 | |
| A | EP 0 448 923 A (AVL PHOTRONICS CORP) 2 October 1991 (1991-10-02) * abstract; figure 1 * --- | 1 | |
| A | DATABASE WPI Week 199213 Derwent Publications Ltd., London, GB; AN 1992-103639 XP002147978 & SU 1 645 893 A (REVUT V I), 30 April 1991 (1991-04-30) * abstract * ----- | 1 | TECHNICAL FIELDS SEARCHED (Int.Cl.7) |

The present search report has been drawn up for all claims

| Place of search | Date of completion of the search | Examiner |
|---|---|---|
| THE HAGUE | 20 September 2000 | Bosma, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 20 0990

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2000

| Patent document cited in search report | Publication date | Patent family member(s) | Publication date |
|---|---|---|---|
| WO 9854354 A | 03-12-1998 | AU 7803098 A | 30-12-1998 |
| | | EP 0988396 A | 29-03-2000 |
| WO 9812348 A | 26-03-1998 | AU 4483997 A | 14-04-1998 |
| | | EP 1021557 A | 26-07-2000 |
| US 5863460 A | 26-01-1999 | AU 2039597 A | 22-10-1997 |
| | | EP 0891540 A | 20-01-1999 |
| | | WO 9737210 A | 09-10-1997 |
| | | US 6074607 A | 13-06-2000 |
| WO 9636875 A | 21-11-1996 | AU 5641996 A | 29-11-1996 |
| | | CA 2149508 A | 16-11-1996 |
| | | CA 2195108 A | 21-11-1996 |
| EP 0448923 A | 02-10-1991 | AT 132537 T | 15-01-1996 |
| | | DE 69024631 D | 15-02-1996 |
| | | DE 69024631 T | 19-09-1996 |
| | | US 5372936 A | 13-12-1994 |
| SU 1645893 A | 30-04-1991 | NONE | |

EPO FORM P0459

For more details about this annex : see Official Journal of the European Patent Office, No. 12/82